# EUROPEAN PATENT APPLICATION

(11) **EP 4 306 647 A1**
(43) Date of publication of application: **17.01.2024**
(21) Application number: 22767207.8
(22) Date of filing: 09.03.2022
(51) Int. Cl.: C12P 21/02, C07K 14/47, C07K 16/18, C07K 19/00, C12N 5/10, C12N 15/12, C12N 15/13, C12N 15/62, C12N 15/63

(54) **METHOD FOR PRODUCING FUSION PROTEIN**

(30) Priority: 10.03.2021 JP 2021038368
(71) Applicant: Sumitomo Pharma Co., Ltd., Osaka 541-0045 (JP)
(72) Inventor: NAGATA, Hidetaka, Osaka-shi, Osaka 554-0022 (JP); LIN, WenLien, Osaka-shi, Osaka 554-0022 (JP); ASADA, Reiko, Osaka-shi, Osaka 554-0022 (JP); TAKIKAWA, Kenji, Osaka-shi, Osaka 541-0045 (JP)
(74) Representative: J A Kemp LLP
(86) International application number: PCT/JP2022/010398
(87) International publication number: WO 2022/191253

(57) **Abstract**

A method for producing a fusion protein containing BDNF and an anti-transferrin receptor antibody or a fragment thereof, the method including: producing the fusion protein by culturing a transformed mammalian cell containing a gene encoding the fusion protein and a gene encoding an exogeneous chaperone protein in a protein production medium; and collecting the produced fusion protein, wherein the gene encoding the fusion protein contains a nucleotide sequence of a gene encoding the BDNF and a nucleotide sequence of a gene encoding the anti-transferrin receptor antibody or a fragment thereof, and the chaperone protein includes one or more selected from the group consisting of HSP90α, HSP900, CDC37, HSP70, HSP40, HSP60, HSP10, HSP110, and HSP27.

## Description

### TECHNICAL FIELD

The present invention relates to a method for producing a fusion protein.

### BACKGROUND ART

Recombinant proteins are currently used in a wide range of fields. The importance is further increasing because of the recent development of biopharmaceuticals. Recombinant proteins are produced primarily with Escherichia coli, yeasts, insect cells, mammalian cells, or the like as host cells (e.g., Japanese National Patent Publication No. 2007-524381 (PTL 1)). Use of such host cells allows recombinant proteins to be given in large quantities in short times. However, recombinant proteins expressed may be incapable of exerting the original functions possessed by the recombinant proteins in some cases, for example, because the recombinant proteins do not correctly fold or have not undergone posttranslational modification (e.g., addition of a sugar chain) or the like.

In particular, brain-derived neurotrophic factor (BDNF), which is a difficult to express protein (DEP), is a member of the neurotrophin family. A dimer of BDNF is known to specifically bind to a high-affinity BDNF receptor present on the surfaces of target cells (TrkB; Tyrosine receptor kinase B, also called Tropomyosin receptor kinase B or Tropomyosin-related Kinase B), playing an important role in differentiation of cells, functional maintenance, synaptogenesis, and regeneration and repair after damaging in the central nervous system and peripheral nervous system (NPL 2 and NPL 3). Therefore, BDNF is expected to be promising for development of a therapeutic agent against various diseases including neurodegenerative diseases such as Alzheimer's disease, Parkinson's disease, and Huntington's disease, spinal degenerative diseases such as amyotrophic lateral sclerosis, and other diseases such as diabetic neuropathy, cerebral ischemic disease, development disorder, schizophrenia, depression, and Rett syndrome. However, BDNF has been known to be difficult to produce as a recombinant protein in large quantities.

A fusion protein containing BDNF and an anti-transferrin receptor antibody is known to be a fusion protein capable of migrating into the brain because BDNF passes through the blood-brain barrier (PTL 2 and PTL 3), and a method for producing it as a recombinant protein in large quantities has been demanded.

### CITATION LIST

### PATENT LITERATURE

PTL 1: Japanese National Patent Publication No. 2007-524381
PTL 2: WO 2016/208696
PTL 3: WO 2018/124107

### NON PATENT LITERATURE

NPL 1: Lee et al., Journal of Biotechnology 143 (2009) 34-43
NPL 2: Moses v. Chao. Nature Reviews Neuroscience. 4. 299-309 (2003)
NPL 3: Bollen E. Behavioural Brain Research. 257C. 8-12 (2013)

### SUMMARY OF INVENTION

### TECHNICAL PROBLEM

A method for producing a fusion protein containing the BDNF and an anti-transferrin receptor antibody or a fragment thereof in large quantities has been also demanded.

The present invention has been made in view of such circumstances, and an object of the present invention is to provide a method for producing a fusion protein with enhanced production efficiency.

### SOLUTION TO PROBLEM

The present inventors diligently studied to achieve the object to find that co-expression of a gene encoding a fusion protein containing BDNF and an anti-transferrin receptor antibody or a fragment thereof and a gene encoding a specific chaperone protein in a mammalian cell as a host cell results in enhanced production efficiency for the fusion protein, completing the present invention. Specifically, the present invention is as follows.
[1] A method for producing a fusion protein of the present invention is a method for producing a fusion protein containing BDNF and an anti-transferrin receptor antibody or a fragment thereof, the method including:
   producing the fusion protein by culturing a transformed mammalian cell containing a gene encoding the fusion protein and a gene encoding an exogeneous chaperone protein in a protein production medium; and
   collecting the produced fusion protein, wherein
   the gene encoding the fusion protein contains the nucleotide sequence of a gene encoding the BDNF and the nucleotide sequence of a gene encoding the anti-transferrin receptor antibody or a fragment thereof, and
   the chaperone protein includes one or more selected from the group consisting of HSP90α, HSP90β, CDC37, HSP70, HSP40, HSP60, HSP10, HSP110, and HSP27.
[2] The method for producing a fusion protein of the present invention is a method for producing a fusion protein containing the BDNF and the anti-transferrin receptor antibody or a fragment thereof, the method including:
   providing a mammalian cell;
   transforming the mammalian cell with a gene encoding the fusion protein and a gene encoding the chaperone protein;
   producing the fusion protein by culturing the transformed mammalian cell in a protein production medium; and
   collecting the produced fusion protein, wherein
   the gene encoding the fusion protein contains the nucleotide sequence of a gene encoding the BDNF and the nucleotide sequence of a gene encoding the anti-transferrin receptor antibody or a fragment thereof, and
   the chaperone protein includes one or more selected from the group consisting of HSP90α, HSP90β, CDC37, HSP70, HSP40, HSP60, HSP10, HSP110, and HSP27.
[3] It is preferable in the method for producing a fusion protein according to [2] that the transforming the mammalian cell be performed with one or more recombinant protein expression vectors each containing a gene encoding the fusion protein and a gene encoding the chaperone protein.
[4] It is preferable in the method for producing a fusion protein according to [2] that the transforming the mammalian cell be performed by simultaneously or separately bringing one or more recombinant protein expression vectors each containing a gene encoding the fusion protein and one or more expression-enhancing vectors each containing a gene encoding the chaperone protein into contact with the mammalian cell.
[5] The method for producing a fusion protein of the present invention is a method for producing a fusion protein containing the BDNF and the anti-transferrin receptor antibody or a fragment thereof, the method including:
   providing a mammalian cell containing one or more recombinant protein expression vectors each containing a gene encoding the fusion protein;
   transforming the mammalian cell with at least one expression-enhancing vector containing a gene encoding the chaperone protein;
   producing the fusion protein by culturing the transformed mammalian cell in a protein production medium; and
   collecting the produced fusion protein, wherein
   the gene encoding the fusion protein contains the nucleotide sequence of a gene encoding the BDNF and the nucleotide sequence of a gene encoding the anti-transferrin receptor antibody or a fragment thereof, and
   the chaperone protein includes one or more selected from the group consisting of HSP90α, HSP90β, CDC37, HSP70, HSP40, HSP60, HSP10, HSP110, and HSP27.
[6] It is preferable in the method for producing a fusion protein according to [5] that
   the expression-enhancing vector include a first expression-enhancing vector containing a gene encoding a first chaperone protein and a second expression-enhancing vector containing a gene encoding a second chaperone protein, and
   the first chaperone protein be different from the second chaperone protein.
[7] It is preferable that the chaperone protein include either one or both of HSP90α and CDC37.
[8] It is preferable that the BDNF be binding, directly or via a linker peptide, to the anti-transferrin receptor antibody or a fragment thereof.
[9] It is preferable that the linker peptide contain an amino acid sequence selected from the group consisting of Gly, Ser, Gly-Ser, Gly-Gly-Ser, Gly-Gly-Gly-Gly-Ser, Gly-Gly-Gly-Gly-Gly-Ser, Ser-Gly-Gly-Gly-Gly, and any 1 to 10 consecutive amino acid sequences selected from those amino acid sequences.
[10] It is preferable that the BDNF contain an amino acid sequence having a sequence identity of at least 90% with an amino acid sequence set forth in SEQ ID NO: 32.
[11] It is preferable that the fragment of the anti-transferrin receptor antibody be a Fab fragment, a F(ab')₂ fragment, or a F(ab') fragment.
[12] It is preferable that the mammalian cell include one or more selected from the group consisting of a CHO cell, a COS cell, a BHK cell, a HeLa cell, an HEK293 cell, an NS0 cell, and an Sp2/0 cell.
[13] A mammalian cell for recombinant protein production according to the present invention is a mammalian cell for recombinant protein production, containing one or more recombinant protein expression vectors each containing a gene encoding a fusion protein, wherein
   the fusion protein contains BDNF and an anti-transferrin receptor antibody or a fragment thereof,
   the gene encoding the fusion protein contains the nucleotide sequence of a gene encoding the BDNF and the nucleotide sequence of a gene encoding the anti-transferrin receptor antibody or a fragment thereof,
   the mammalian cell for recombinant protein production further contains one or more expression-enhancing vectors each containing a gene encoding a chaperone protein, and
   the chaperone protein includes one or more selected from the group consisting of HSP90α, HSP900, CDC37, HSP70, HSP40, HSP60, HSP10, HSP110, and HSP27.
[14] A kit according to the present invention is a kit for enhancing the production of a fusion protein containing BDNF and an anti-transferrin receptor antibody or a fragment thereof in a mammalian cell, wherein
   the kit includes one or more expression-enhancing vectors each containing a gene encoding a chaperone protein, and
   the chaperone protein includes at least one selected from the group consisting of HSP90α, HSP900, CDC37, HSP70, HSP40, HSP60, HSP10, HSP110, and HSP27.

### ADVANTAGEOUS EFFECT OF INVENTION

The present invention enables providing a method for producing a fusion protein with enhanced production efficiency.

### BRIEF DESCRIPTION OF DRAWINGS

Fig. 1 is a graph representing production levels of a human BDNF-human anti-transferrin receptor antibody Fab fragment in transformed mammalian cells.
Fig. 2 is a graph representing production levels of a human BDNF-human anti-transferrin receptor antibody Fab fragment in transformed mammalian cells.
Fig. 3 is a graph representing viable cell counts of transformed mammalian cells after production of a human BDNF-human anti-transferrin receptor antibody Fab fragment through culture.

### DESCRIPTION OF EMBODIMENTS

The following describes an embodiment of the present invention (hereinafter, occasionally expressed as "the present embodiment"). However, the present embodiment is not limited to the description. Herein, expressions in the format of "A to Z" each indicate the upper limit and lower limit of a range (i.e., A or more and Z or less). In the case that a unit is shown not for A but only for Z, the unit of A and the unit of Z are the same.

A method for producing a fusion protein of the present embodiment is a method for producing a fusion protein containing BDNF and an anti-transferrin receptor antibody or a fragment thereof, the method including:
producing the fusion protein by culturing a transformed mammalian cell containing a gene encoding the fusion protein and a gene encoding an exogeneous chaperone protein in a protein production medium; and
collecting the produced fusion protein, wherein
the gene encoding the fusion protein contains the nucleotide sequence of a gene encoding the BDNF and the nucleotide sequence of a gene encoding the anti-transferrin receptor antibody or a fragment thereof, and
the chaperone protein includes one or more selected from the group consisting of HSP90α, HSP90β, CDC37, HSP70, HSP40, HSP60, HSP10, HSP110, and HSP27.

In an aspect of the present embodiment, the transformed mammalian cell can be obtained with a method including:
providing a mammalian cell containing one or more recombinant protein expression vectors each containing a gene encoding the fusion protein; and
transforming the mammalian cell with at least one expression-enhancing vector containing a gene encoding a chaperone protein.
The details will be described in "Method for producing fusion protein (1)" shown later.

In another aspect of the present embodiment, the transformed mammalian cell can be obtained with a method including:
providing a mammalian cell; and
transforming the mammalian cell with a gene encoding the fusion protein and a gene encoding a chaperone protein.
The details will be described in "Method for producing fusion protein (2)" shown later.

### <<Method for producing fusion protein (1)>>

A first method for producing a fusion protein of the present embodiment is a method for producing a fusion protein containing BDNF and an anti-transferrin receptor antibody or a fragment thereof, the method including:
providing a mammalian cell containing one or more recombinant protein expression vectors each containing a gene encoding the fusion protein;
transforming the mammalian cell with at least one expression-enhancing vector containing a gene encoding a chaperone protein;
producing the fusion protein by culturing the transformed mammalian cell in a protein production medium; and
collecting the produced fusion protein, wherein
the gene encoding the fusion protein contains the nucleotide sequence of a gene encoding the BDNF and the nucleotide sequence of a gene encoding the anti-transferrin receptor antibody or a fragment thereof (hereinafter, occasionally referred to as "additional protein"), and
the chaperone protein includes one or more selected from the group consisting of HSP90α, HSP90β, CDC37, HSP70, HSP40, HSP60, HSP10, HSP110, and HSP27. The following describes the first method in detail.

### <Providing mammalian cell containing recombinant protein expression vector>

In this step, a mammalian cell containing one or more recombinant protein expression vectors each containing a gene encoding the fusion protein is provided. The gene encoding the fusion protein contains the nucleotide sequence of a gene encoding the BDNF and the nucleotide sequence of a gene encoding the additional protein.

### (Fusion protein)

The "fusion protein" in the present embodiment contains BDNF and an anti-transferrin receptor antibody or a fragment thereof (hereinafter, occasionally referred to as "additional protein"). In an aspect of the present embodiment, the fusion protein may consist only of BDNF and an additional protein. In another aspect of the present embodiment, the fusion protein may be composed of BDNF, the additional protein, and a linker peptide linking the BDNF and the additional protein. That is, it is preferable that the BDNF be binding, directly or via a linker peptide, to the anti-transferrin receptor antibody or a fragment thereof.

The linker peptide is not limited as long as it has a known amino acid sequence. Examples of the amino acid sequence of the linker peptide include an amino acid sequence with five repetitions of an amino acid sequence represented by "GGGGS" (SEQ ID NO: 76) in one letter amino acid codes (SEQ ID NO: 75). Another example is a peptide linker with two to four consecutive H4 linkers represented by "EAAAAK" (SEQ ID NO: 77). In an aspect of the present embodiment, it is preferable that the linker peptide contain an amino acid sequence selected from the group consisting of Gly, Ser, Gly-Ser, Gly-Gly-Ser, Gly-Gly-Gly-Gly-Ser, Gly-Gly-Gly-Gly-Gly-Ser, Ser-Gly-Gly-Gly-Gly, and an amino acid sequence consisting of any 1 to 10 consecutive, identical or different amino acid sequences selected from those amino acid sequences.

In the fusion protein, the BDNF and the additional protein may be disposed in the N-terminal side and in the C-terminal side, respectively. In the fusion protein, the additional protein and the BDNF may be disposed in the N-terminal side and in the C-terminal side, respectively. That is, the BDNF may be binding, directly or via a linker peptide, to the C-terminal side or N-terminal side of a heavy chain and/or light chain of the anti-transferrin receptor antibody or a fragment thereof.

Specific examples of the fusion protein include a fusion protein (a fusion protein of BDNF and an anti-transferrin receptor antibody) described in WO 2016/208696 (PTL 2) or WO 2018/124107 (PTL 3).

### (BDNF)

Here, the BDNF is a known protein discovered by Barde et al. in 1982 and cloned by Jones et al. in 1990 (EMBO J, (1982) 1: 549-553, Proc. Natl. Acad. Sci. USA (1990) 87: 8060-8064). The term BDNF encompasses: a mature BDNF, which exerts the functions in vivo; a BDNF precursor, a premature form of BDNF (also referred to as "BDNF pro-form"); and a precursor of the BDNF precursor (also referred to as "BDNF pre-pro-form"), which is formed by adding a signal peptide to the N terminus of the BDNF precursor. Specifically, the BDNF is first formed as a BDNF pre-pro-form from the gene transcription product, and a signal peptide is cleaved from the BDNF pre-pro-form to leave a BDNF pro-form. Thereafter, the 110 N-terminal amino acid residues are cleaved from the BDNF pro-form to leave a mature BDNF.

### (Additional protein)

In the present embodiment, the term "additional protein" refers to an anti-transferrin receptor antibody or an anti-transferrin receptor antibody fragment constituting the fusion protein together with the BDNF. The additional protein may be a monomer, or a dimer composed of two subunits, or a multimer composed of multiple subunits. Examples of the antibody fragment include a Fab fragment, a F(ab')₂ fragment, and a F(ab') fragment, each of which consists of a heavy chain (H chain) fragment of an antibody and a light chain (L chain) fragment of an antibody, an Fc fragment in which the Fab fragment is added to an antibody constant region, a single-chain antibody (scFv), and a bispecific antibody (diabody).

### (Recombinant protein expression vector)

In the present embodiment, the term "recombinant protein expression vector" refers to a DNA construct in which a gene encoding a target recombinant protein has been introduced in such a manner that the gene can be expressed in a host cell. The term "recombinant protein" refers to an exogenous protein for the host cell. In the present embodiment, the target recombinant protein is the fusion protein. That is, the recombinant protein expression vector contains a gene encoding the fusion protein. The gene encoding the fusion protein contains the nucleotide sequence of a gene encoding the BDNF and the nucleotide sequence of a gene encoding the additional protein.

The additional protein is a dimer, the gene encoding the fusion protein may be composed of a first gene containing the nucleotide sequence of a gene encoding the BDNF and the nucleotide sequence of a gene encoding the first subunit constituting the additional protein, and a second gene containing the nucleotide sequence of a gene encoding the second subunit constituting the additional protein. In this case, the recombinant protein expression vector may be composed of a first recombinant protein expression vector containing the first gene and a second recombinant protein expression vector containing the second gene. Alternatively, the recombinant protein expression vector may contain both the first gene and the second gene. Even in the case that the additional protein is a multimer, design of the gene encoding the fusion protein and design of the recombinant protein expression vector can be carried out in the same manner as in the aforementioned case of dimer.

In the present embodiment, the nucleotide sequence of the gene encoding the BDNF may be a wild-type nucleotide sequence, and may be a nucleotide sequence formed by introducing one or more mutations into the wild-type nucleotide sequence. Specifically, the nucleotide sequence of the gene encoding the BDNF may be:
(A) a nucleotide sequence having a sequence identity of 90% or more and 100% or less with the wild-type nucleotide sequence encoding the BDNF;
(B) a nucleotide sequence formed by deleting, substituting, inserting, or adding one or several nucleotides for the wild-type nucleotide sequence encoding the BDNF;
(C) a nucleotide sequence hybridizable with an oligonucleotide having a nucleotide sequence complementary to the wild-type nucleotide sequence encoding the BDNF under stringent conditions;
(D) a nucleotide sequence encoding an amino acid sequence having a sequence identity of 90% or more and 100% or less with the wild-type amino acid sequence of the BDNF; or
(E) a nucleotide sequence encoding an amino acid sequence formed by deleting, substituting, inserting, or adding one or several amino acid residues for the wild-type amino acid sequence of the BDNF, and
the nucleotide sequence of the gene encoding the BDNF may be a nucleotide sequence encoding a protein retaining the original functions of the BDNF.

Here, the phrase "retaining the original functions" means having equivalent functions to wild-type BDNF. The term "equivalent functions" means that the characteristics are qualitatively the same, for example, in a physiological sense or in a pharmacological sense, and the degree of functioning (e.g., approximately 0.1 to approximately 10 times, preferably 0.5 to 2 times) or the quantitative factors such as the molecular weight of the protein may be different. A protein that can be recognized by an antibody that specifically recognizes a protein having functions possessed by wild-type BDNF such as (1) binding affinity to the BDNF receptor (TrkB), (2) phosphorylation activity for the BDNF receptor, (3) growth-promoting effect for neurons, (4) survival-and-maintenance effect for neurons, and (5) neurite extension effect for neurons, or (6) consisting of an amino acid sequence set forth in SEQ ID NO: 74 is regarded as a "protein having equivalent functions" to BDNF.

In the case that the BDNF is a BDNF pre-pro-form, the expression "original functions" indicates, for example, being capable of forming a BDNF pro-form. In the case that the BDNF is a BDNF pro-form, the expression "original functions" indicates, for example, being capable of forming a mature BDNF, or having binding affinity to the p75 receptor.

In the present embodiment, "sequence identity" means the proportion (%) of identical nucleotides to the total nucleotides of an overlapping nucleotide sequence in optimum alignment given by aligning two nucleotide sequences with a mathematical algorithm known in the art (preferably, the algorithm allows introduction of a gap into one or both of sequences to be considered for optimum alignment). Those skilled in the art could check the "sequence identity" of a nucleotide sequence with ease. For example, NCBI BLAST (National Center for Biotechnology Information Basic Local Alignment Search Tool) can be used. The sequence identity of an amino acid sequence can also be checked with the same method as described.

The nucleotide sequence of the gene encoding the BDNF may have a sequence identity of 95% or more and 100% or less, or a sequence identity of 98% or more and 100% or less, or a sequence identity of 100% with the wild-type nucleotide sequence encoding the BDNF.

In the present embodiment, examples of the "nucleotide sequence formed by deleting, substituting, inserting, or adding one or several nucleotides" include a nucleotide sequence having a sequence identity of 80% or more, 85% or more, 90% or more, 95% or more, 97% or more, 98% or more, or 99% or more with the nucleotide sequence before deletion, substitution, insertion, or addition as a result of deletion, substitution, insertion, or addition. Regarding the specific number for "one or several nucleotides", any one of such deletion, substitution, insertion, and addition may be present at one position, two positions, three positions, four positions, or five positions, and two or more of such deletion, substitution, insertion, and addition may occur in combination.

In the present embodiment, the term "stringent conditions" refers to conditions involving incubating in a solution containing 6 × SSC (composition of 1 × SSC: 0.15 M NaCl, 0.015 M sodium citrate, pH 7.0), 0.5% SDS, 5 × Denhardt's solution, 100 µg/mL modified salmon sperm DNA, and 50% (v/v) formamide at room temperature for 12 hours, and further washing with 0.5 × SSC at a temperature of 50°C or more. Moreover, the term "stringent conditions" also encompasses more stringent conditions, for example, more severe conditions such as incubating at 45°C or 60°C for 12 hours, washing with 0.2 × SSC or 0.1 × SSC, and washing under a temperature condition of 60°C or 65°C or more in washing.

In an aspect of the present embodiment, the nucleotide sequence of the gene encoding the BDNF may be a nucleotide sequence subjected to optimization of codons with considering codon usage frequencies in the mammalian cell into which the gene is to be introduced. The optimization of codons is performed, for example, as follows. Specifically, the optimization of codons can be performed by using an algorithm capable of optimizing transcription, translational effects, and folding formation, as typified by Codon W (e.g., see http://codonw.sourceforge.net/index.html).

In the present embodiment, examples of the nucleotide sequence of a gene encoding BDNF include a nucleotide sequence encoding the BDNF pre-pro-form described above, a nucleotide sequence encoding the BDNF pro-form, and a nucleotide sequence encoding the mature BDNF. Examples of the nucleotide sequence encoding the BDNF pre-pro-form include nucleotide sequences set forth in SEQ ID NO: 71 (GenBank No. NM_ 170735.6, human-derived wild nucleotide sequences). Examples of the nucleotide sequence encoding the BDNF pro-form include a nucleotide sequence encoding the amino acid sequence of a BDNF pro-form formed by removing a signal peptide corresponding to the 18 N-terminal amino acid residues of the BDNF pre-pro-form (e.g., SEQ ID NO: 31). Examples of the nucleotide sequence encoding the mature BDNF include a nucleotide sequence encoding a mature BDNF formed by removing the 110 N-terminal amino acid residues of the BDNF pro-form (e.g., SEQ ID NO: 73). Here, the signal peptide in the BDNF pre-pro-form may be a signal peptide possessed by the wild BDNF pre-pro-form, or a signal peptide derived from another protein (e.g., a signal peptide consisting of an amino acid sequence set forth in SEQ ID NO: 36).

The amino acid sequence of the BDNF may have a sequence identity of 95% or more and 100% or less, or a sequence identity of 98% or more and 100% or less, or a sequence identity of 100% with the wild-type amino acid sequence of the BDNF.

In the present embodiment, examples of the "amino acid sequence formed by deleting, substituting, inserting, or adding one or several amino acid residues" include an amino acid sequence having a sequence identity of 80% or more, 85% or more, 90% or more, 95% or more, 97% or more, 98% or more, or 99% or more with the amino acid sequence before deletion, substitution, insertion, or addition as a result of deletion, substitution, insertion, or addition. Regarding the specific number for "one or several amino acid residues", any one of such deletion, substitution, insertion, and addition may be present at one position, two positions, three positions, four positions, or five positions, and two or more of such deletion, substitution, insertion, and addition may occur in combination.

In the present embodiment, examples of the amino acid sequence of BDNF include the amino acid sequence of the BDNF pre-pro-form described above, the amino acid sequence of the BDNF pro-form, and the amino acid sequence of the mature BDNF. Examples of the amino acid sequence of the BDNF pre-pro-form include an amino acid sequence set forth in SEQ ID NO: 72 (GenBank No. NP_733931). Examples of the amino acid sequence of the BDNF pro-form include an amino acid sequence formed by removing the N-terminal signal peptide of the BDNF pre-pro-form (e.g., SEQ ID NO: 32). Examples of the amino acid sequence of the mature BDNF include an amino acid sequence formed by removing the 110 N-terminal amino acid residues of the BDNF pro-form (e.g., SEQ ID NO: 74).

In the present embodiment, the nucleotide sequence of the gene encoding the additional protein may be a wild-type nucleotide sequence, or a nucleotide sequence formed by introducing one or more mutations into the wild-type nucleotide sequence. Specifically, the nucleotide sequence of the gene encoding the additional protein may be:
(A) a nucleotide sequence having a sequence identity of 90% or more and 100% or less with the wild-type nucleotide sequence encoding the additional protein;
(B) a nucleotide sequence formed by deleting, substituting, inserting, or adding one or several nucleotides for the wild-type nucleotide sequence encoding the additional protein;
(C) a nucleotide sequence hybridizable with an oligonucleotide having a nucleotide sequence complementary to the wild-type nucleotide sequence encoding the additional protein under stringent conditions;
(D) a nucleotide sequence encoding an amino acid sequence having a sequence identity of 90% or more and 100% or less with the wild-type amino acid sequence of the additional protein; or
(E) a nucleotide sequence encoding an amino acid sequence formed by deleting, substituting, inserting, or adding one or several amino acid residues for the wild-type amino acid sequence of the additional protein, and
the nucleotide sequence of the gene encoding the additional protein may be a nucleotide sequence encoding a protein retaining the original functions of the additional protein.

In the case that the additional protein is a dimer or a multimer, the aforementioned matters are applied to each of the genes encoding the subunits constituting the additional protein.

The nucleotide sequence of the gene encoding the additional protein may have a sequence identity of 95% or more and 100% or less, or a sequence identity of 98% or more and 100% or less, or a sequence identity of 100% with the wild-type nucleotide sequence encoding the additional protein.

In an aspect of the present embodiment, the nucleotide sequence of the gene encoding the additional protein may be a nucleotide sequence subjected to optimization of codons with considering codon usage frequencies in the mammalian cell into which the gene is to be introduced. The optimization of codons is performed, for example, with the method described above.

In the present embodiment, in the case that the additional protein is a Fab fragment, examples of the nucleotide sequence of a gene encoding a heavy chain fragment (first subunit) of an antibody include a nucleotide sequence set forth in SEQ ID NO: 37. Examples of the nucleotide sequence of a gene encoding a light chain fragment (second subunit) of an antibody include a nucleotide sequence set forth in SEQ ID NO: 41.

The amino acid sequence of the additional protein may have a sequence identity of 95% or more and 100% or less, or a sequence identity of 98% or more and 100% or less, or a sequence identity of 100% with the wild-type (in this case, a Fab fragment containing a protein encoded by the nucleotide sequence set forth in SEQ ID NO: 37 and a protein encoded by the nucleotide sequence set forth in SEQ ID NO: 41) amino acid sequence of the additional protein.

Regarding the additional protein, a Fab fragment of an additional protein containing a heavy chain fragment set forth in SEQ ID NO: 38 and a light chain fragment set forth in SEQ ID NO: 42, and an additional protein having the above sequence identity are described in PTL 3.

In the present embodiment, in the case that the additional protein is a Fab fragment, examples of the amino acid sequence of a heavy chain fragment (first subunit) of an antibody include an amino acid sequence set forth in SEQ ID NO: 38. Examples of the amino acid sequence of a light chain fragment (second subunit) of an antibody include an amino acid sequence set forth in SEQ ID NO: 42.

A Fab fragment of an additional protein containing the heavy chain fragment set forth in SEQ ID NO: 38 and the light chain fragment set forth in SEQ ID NO: 42 is described in PTL 3.

The recombinant protein expression vector contains not only the gene encoding the fusion protein but also a promoter sequence (e.g., a cytomegalovirus (CMV) promoter, a thymidine kinase (TK) promoter of herpes simplex virus (HSV), an SV40 promoter, an EF-1 promoter, an actin promoter, a β globulin promoter, and an enhancer), a Kozak sequence, a terminator sequence, and an mRNA-stabilizing sequence. In an aspect of the present embodiment, the recombinant protein expression vector may further contain one or more selected from the group consisting of an origin of replication, an enhancer sequence, a signal sequence, a selection marker gene such as a drug resistance gene (e.g., a resistance gene against a drug such as ampicillin, tetracycline, kanamycin, chloramphenicol, neomycin, hygromycin, puromycin, and Zeocin), and a gene encoding a fluorescent protein such as GFP.

The recombinant protein expression vector is not limited as long as the advantageous effects of the present invention are exerted, and may be, for example, a plasmid vector or a viral vector. In an aspect of the present embodiment, it is preferable that the recombinant protein expression vector be a plasmid vector.

Examples of the plasmid vector include a pcDNA3.1(+) vector, a pEGF-BOS vector, a pEF vector, a pCDM8 vector, a pCXN vector, a pCI vector, an episomal vector, and a transposon vector. In an aspect of the present embodiment, it is preferable that the plasmid vector be a pcDNA3.1(+) vector.

Examples of the viral vector include a lentiviral vector, an adenoviral vector, an adeno-associated virus vector, a Sendai virus vector, and a mammalian expression baculoviral vector. Examples include pLenti4/V5-GW/lacZ, pLVSIN-CMV, pLVSIN-EF1α, pAxcwit2, pAxEFwit2, pAAV-RCS, a pSeV vector, pFastBacMam, and pFastBacMam2.0 (VSV-G).

### (Mammalian cell)

In the present embodiment, the term "mammalian cell" refers to a cell derived from a mammal. Examples of the mammal include a human, a hamster (e.g., a Chinese hamster), a mouse, a rat, and a green monkey. The mammalian cell may be an immortalized cell.

The mammalian cell is not limited as long as it is used as a host cell for expression of the recombinant protein. Examples of such a mammalian cell include a CHO cell (a cell line derived from the ovary of a Chinese hamster), a COS cell (a cell line derived from the kidney of an African green monkey), a BHK cell (a cell line derived from the kidney of a baby hamster), a HeLa cell (a cell line derived from cervical cancer of a human), an HEK293 cell (a cell line derived from the kidney of a human embryo), an NS0 cell (a cell line derived from myeloma of a mouse), and an Sp2/0 cell (a cell line derived from myeloma of a mouse). Specifically, it is preferable that the mammalian cell include one or more selected from the group consisting of a CHO cell, a COS cell, a BHK cell, a HeLa cell, an HEK293 cell, an NS0 cell, and an Sp2/0 cell.

### <Transforming mammalian cell with expression-enhancing vector>

In this step, the mammalian cell is transformed with at least one expression-enhancing vector containing a gene encoding a chaperone protein.

### (Chaperone protein)

In the present embodiment, the term "chaperone protein" refers to a protein that assists the fusion protein in correctly folding to attain the original functions. The "original functions of the fusion protein" refer to the original functions possessed by the BDNF and additional protein constituting the fusion protein. The chaperone protein includes one or more selected from the group consisting of HSP90α, HSP900, CDC37 (Cell Division Cycle 37, HSP90 cochaperone), HSP70, HSP40, HSP60, HSP10, HSP110, and HSP27. Here, "HSP" is an abbreviation of heat shock protein. In an aspect of the present embodiment, it is preferable that the chaperone protein include any one of HSP90α, HSP90β, HSP40, and CDC37, or include both HSP90α, HSP90β, or HSP40 and CDC37.

In another aspect of the present embodiment, it is preferable that the chaperone protein include either one or both of HSP90α and CDC37.

In an aspect of the present mode of implementation, the animal species from which the chaperone protein is derived may be the same as or different from the animal species from which the cysteine knot protein is derived.

In an aspect of the present mode of implementation, the animal species from which the chaperone protein is derived may be the same as or different from the animal species from which the host cell is derived.

In an aspect of the present mode of implementation, it is preferable that the animal species from which the chaperone protein is derived be the same as either one of the animal species from which the cysteine knot protein is derived or the animal species from which the host cell is derived.

In an aspect of the present embodiment, the chaperone protein may be a human-derived chaperone protein, or a Chinese hamster-derived chaperone protein. The chaperone protein may be preferably a human-derived chaperone protein.

In an aspect of the present embodiment, in the case that the fusion protein contains BDNF and a Fab fragment, it is preferable that the chaperone protein include one or more selected from the group consisting of HSP90α, HSP90β, HSP60, HSP10, HSP70, and HSP27.

### (Expression-enhancing vector)

In the present embodiment, the term "expression-enhancing vector" refers to a DNA construct in which a gene encoding the chaperone protein has been introduced in such a manner that the gene can be expressed in a host cell.

In the present embodiment, the nucleotide sequence of the gene encoding the chaperone protein may be a wild-type nucleotide sequence, or a nucleotide sequence formed by introducing one or more mutations into the wild-type nucleotide sequence. Specifically, the nucleotide sequence of the gene encoding the chaperone protein may be:
(A) a nucleotide sequence having a sequence identity of 90% or more and 100% or less with the wild-type nucleotide sequence encoding the chaperone protein;
(B) a nucleotide sequence formed by deleting, substituting, inserting, or adding one or several nucleotides for the wild-type nucleotide sequence encoding the chaperone protein;
(C) a nucleotide sequence hybridizable with an oligonucleotide having a nucleotide sequence complementary to the wild-type nucleotide sequence encoding the chaperone protein under stringent conditions;
(D) a nucleotide sequence encoding an amino acid sequence having a sequence identity of 90% or more and 100% or less with the wild-type amino acid sequence of the chaperone protein; or
(E) a nucleotide sequence encoding an amino acid sequence formed by deleting, substituting, inserting, or adding one or several amino acid residues for the wild-type amino acid sequence of the chaperone protein, and
the nucleotide sequence of the gene encoding the chaperone protein may be a nucleotide sequence encoding a protein that assists the BDNF in correctly folding to attain the original functions.

The nucleotide sequence of the gene encoding the chaperone protein may have a sequence identity of 95% or more and 100% or less, or a sequence identity of 98% or more and 100% or less, or a sequence identity of 100% with the wild-type nucleotide sequence encoding the chaperone protein.

In an aspect of the present embodiment, the nucleotide sequence of the gene encoding the chaperone protein may be a nucleotide sequence subjected to optimization of codons with considering codon usage frequencies in the mammalian cell into which the gene is to be introduced. The optimization of codons is performed, for example, with the method described above.

In the present embodiment, examples of the nucleotide sequence of a gene encoding HSP90α include nucleotide sequences set forth in SEQ ID NO: 45 (GenBank No. NM_001017963, human-derived wild nucleotide sequence), SEQ ID NO: 47 (GenBank No. NM_005348, human-derived wild nucleotide sequence), SEQ ID NO: 1, SEQ ID NO: 3, SEQ ID NO: 49 (GenBank No. NM_001246821, Chinese hamster-derived wild nucleotide sequence), and SEQ ID NO: 5.

Examples of the nucleotide sequence of a gene encoding HSP90β include nucleotide sequences set forth in SEQ ID NO: 53 (GenBank No. NM_001271970, human-derived wild nucleotide sequence), SEQ ID NO: 55 (GenBank No. NM_001271971, human-derived wild nucleotide sequence), SEQ ID NO: 51 (GenBank No. NM_001271972, human-derived wild nucleotide sequence), SEQ ID NO: 7, SEQ ID NO: 9, SEQ ID NO: 11, SEQ ID NO: 57 (GenBank No. XM_003501668.2, Chinese hamster-derived wild nucleotide sequence), and SEQ ID NO: 13.

Examples of the nucleotide sequence of a gene encoding CDC37 include nucleotide sequences set forth in SEQ ID NO: 59 (GenBank No. NM_007065, human-derived wild nucleotide sequence), SEQ ID NO: 15, SEQ ID NO: 61 (GenBank No. XM_003499737, Chinese hamster-derived wild nucleotide sequence), and SEQ ID NO: 17.

Examples of the nucleotide sequence of a gene encoding HSP60 include nucleotide sequences set forth in SEQ ID NO: 63 (GenBank No. NM_199440, human-derived wild nucleotide sequence) and SEQ ID NO: 19.

Examples of the nucleotide sequence of a gene encoding HSP40 include nucleotide sequences set forth in SEQ ID NO: 65 (GenBank No. NM_001539, human-derived wild nucleotide sequence) and SEQ ID NO: 21.

Examples of the nucleotide sequence of a gene encoding HSP10 include nucleotide sequences set forth in SEQ ID NO: 67 (GenBank No. NM_002157, human-derived wild nucleotide sequence) and SEQ ID NO: 23.

Examples of the nucleotide sequence of a gene encoding HSP110 include nucleotide sequences set forth in SEQ ID NO: 69 (GenBank No. NM_006644, human-derived wild nucleotide sequence) and SEQ ID NO: 25.

Examples of the nucleotide sequence of a gene encoding HSP70 include a CHO-derived wild nucleotide sequence described in Journal of Biotechnology 143 (2009) 34-43 and a nucleotide sequence set forth in SEQ ID NO: 27.

Examples of the nucleotide sequence of a gene encoding HSP27 include a CHO-derived wild nucleotide sequence described in Journal of Biotechnology 143 (2009) 34-43 and a nucleotide sequence set forth in SEQ ID NO: 29.

The amino acid sequence of the chaperone protein may have a sequence identity of 95% or more and 100% or less, or a sequence identity of 98% or more and 100% or less, or a sequence identity of 100% with the wild-type amino acid sequence of the chaperone protein.

In the present embodiment, examples of the amino acid sequence of HSP90α include amino acid sequences set forth in SEQ ID NO: 2 (GenBank No. NP_001017963), SEQ ID NO: 4 (GenBank No. NP_005339), and SEQ ID NO: 6 (GenBank No. NP_001233750).

Examples of the amino acid sequence of HSP90β include amino acid sequences set forth in SEQ ID NO: 8 (GenBank No. NP_001258899), SEQ ID NO: 10 (GenBank No. NP_001258900), SEQ ID NO: 12 (GenBank No. NP_001258901), and SEQ ID NO: 14 (GenBank No. XP _003501716).

Examples of the amino acid sequence of CDC37 include amino acid sequences set forth in SEQ ID NO: 16 (Genbank No. NP_008996) and SEQ ID NO: 18 (GenBank No. XP_003499785).

Examples of the amino acid sequence of HSP60 include an amino acid sequence set forth in SEQ ID NO: 20 (GenBank No. NP_955472).

Examples of the amino acid sequence of HSP40 include an amino acid sequence set forth in SEQ ID NO: 22 (GenBank No. NP_001530).

Examples of the amino acid sequence of HSP10 include an amino acid sequence set forth in SEQ ID NO: 24 (GenBank No. NP_002148).

Examples of the amino acid sequence of HSP 110 include an amino acid sequence set forth in SEQ ID NO: 26 (GenBank No. NP_006635).

Examples of the amino acid sequence of HSP70 include an amino acid sequence set forth in SEQ ID NO: 28 (described in Journal of Biotechnology 143 (2009) 34-43).

Examples of the amino acid sequence of HSP27 include an amino acid sequence set forth in SEQ ID NO: 30 (described in Journal of Biotechnology 143 (2009) 34-43).

The expression-enhancing vector contains not only the gene encoding the chaperone protein but also a promoter sequence (e.g., a cytomegalovirus (CMV) promoter, a thymidine kinase (TK) promoter of herpes simplex virus (HSV), an SV40 promoter, an EF-1 promoter, an actin promoter, a β globulin promoter, and an enhancer), a Kozak sequence, a terminator sequence, and an mRNA-stabilizing sequence. In an aspect of the present embodiment, the expression-enhancing vector may further contain one or more selected from the group consisting of an origin of replication, an enhancer sequence, a signal sequence, a selection marker gene such as a drug resistance gene (e.g., a resistance gene against a drug such as ampicillin, tetracycline, kanamycin, chloramphenicol, neomycin, hygromycin, puromycin, and Zeocin), and a gene encoding a fluorescent protein such as GFP.

The expression-enhancing vector is not limited as long as the advantageous effects of the present invention are exerted, and may be, for example, a plasmid vector or a viral vector. In an aspect of the present embodiment, it is preferable that the expression-enhancing vector be a plasmid vector.

Examples of the plasmid vector include a pcDNA3.1(+) vector, a pEGF-BOS vector, a pEF vector, a pCDM8 vector, a pCXN vector, a pCI vector, an episomal vector, and a transposon vector. In an aspect of the present embodiment, it is preferable that the plasmid vector be a pcDNA3.1(+) vector.

Examples of the viral vector include a lentiviral vector, an adenoviral vector, an adeno-associated virus vector, a Sendai virus vector, and a mammalian expression baculoviral vector. Examples include pLenti4/V5-GW/lacZ, pLVSIN-CMV, pLVSIN-EF1α, pAxcwit2, pAxEFwit2, pAAV-RCS, a pSeV vector, pFastBacMam, and pFastBacMam2.0 (VSV-G).

In the present embodiment, design of a gene encoding the fusion protein, acquisition of a gene fragment encoding the BDNF, acquisition of a gene fragment encoding the additional protein, acquisition of a gene fragment encoding the chaperone protein, and construction of the plasmid vector can be carried out according to techniques conventionally used in the fields of molecular biology, biotechnology, and genetic engineering (e.g., Sambrook et al. "Molecular Cloning-A Laboratory Manual, second edition 1989"). Examples of the host cell to be used for preparing the plasmid vector include Escherichia coli, which is commonly used in the art.

In the present step, the mammalian cell can be suitably transformed with at least one expression-enhancing vector, whereas the mammalian cell may be transformed with a plurality of expression-enhancing vectors. Here, the mammalian cell is a mammalian cell containing one or more recombinant protein expression vectors each containing a gene encoding the fusion protein, provided in the previous step. That is, in an aspect of the present embodiment, it is preferable that the expression-enhancing vector include a first expression-enhancing vector containing a gene encoding a first chaperone protein and a second expression-enhancing vector containing a gene encoding a second chaperone protein, and the first chaperone protein be different from the second chaperone protein. In this case, it is more preferable that the first chaperone protein be HSP90α and the second chaperone protein be CDC37.

Alternatively, the mammalian cell may be transformed with an expression-enhancing vector containing two or more genes each encoding a chaperone protein.

### (Transformation with expression-enhancing vector)

In the present embodiment, any known method can be used for transforming with the expression-enhancing vector without limitation as long as the advantageous effects of the present invention are exerted (e.g., Sambrook et al. "Molecular Cloning-A Laboratory Manual, second edition 1989"). Examples of known transformation methods include a lipofection method, a calcium phosphate method, a DEAE dextran method, an electroporation method, a polyethyleneimine method, and a polyethylene glycol method. The transformation may be performed with commercially available kit. Examples of such kits include a Gibco (TM) Expi (TM) Expression System (Cat. No. A29133) manufactured by ThermoFisher Scientific K.K.

In the case of the lipofection method, 3 µg to 30 µg of an expression vector is used for a cell density of 1 × 10⁶ cells/mL to 9 × 10⁶ cells/mL, for example. For example, 20 µg in total of the expression-enhancing vector is used for cells (6 × 10⁶ cells/mL) contained in a 25-mL container.

### <Producing fusion protein>

In this step, the fusion protein is produced by culturing the transformed mammalian cell in a protein production medium.

Methods for producing recombinant proteins in large quantities by using Escherichia coli as host cells have been known so far; however, a method for producing a cysteine knot protein (e.g., BDNF), which is a difficult to express protein, or a fusion protein containing the cysteine knot protein in large quantities as a soluble fraction such that the conformation that exhibits the functions is retained, by using mammalian cells as host cells has not been known yet. The method for producing a fusion protein according to the present embodiment gives enhanced production efficiency for the fusion protein by co-expressing a gene encoding the fusion protein and a gene encoding the specific chaperone protein in the mammalian cell.

In culturing the transformed mammalian cell, the composition of the medium, the pH of the medium, the glucose concentration, the culture temperature, and the culture time, and other conditions such as the amounts of usage and time of usage of expression-inducing factors are appropriately adjusted so that the fusion protein and the chaperone protein can be efficiently expressed.

The protein production medium to be used for culturing the transformed mammalian cell is not limited as long as it is a known medium suitable for protein production, and may be a solid medium or a liquid medium. It is preferable that the protein production medium be a liquid medium. Examples of the protein production medium include Dulbecco's modified Eagle medium (DMEM), Eagle minimal essential medium (MEM), Roswell Park Memorial Institute medium 1640 (RPMI1640), Iscove's modified Dulbecco's medium (IMDM), F10 medium, F12 medium, DMEM/F12, FreeStyle 293 expression medium, and Freestyle CHO medium. The protein production medium may contain fetal calf serum (FCS). The protein production medium may be a serum-free medium.

### <Collecting fusion protein>

In this step, the produced fusion protein is collected. The present step includes collecting the produced fusion protein from the culture supernatant after the completion of culture. For example, after the completion of culture, the fusion protein purified to have high purity can be obtained by treating the resulting culture supernatant with various purification methods.

At least one may be selected as a purification method from, for example, heat treatment and salting-out for the culture supernatant, and various chromatographies including anion-exchange chromatography, gel filtration chromatography, hydrophobic chromatography, hydroxyapatite chromatography, and affinity chromatography.

### <<Method for producing fusion protein (2)>>

A second method for producing a fusion protein of the present embodiment is a method for producing a fusion protein containing BDNF and an anti-transferrin receptor antibody or a fragment thereof, the method including:
providing a mammalian cell;
transforming the mammalian cell with a gene encoding the fusion protein and a gene encoding a chaperone protein;
producing the fusion protein by culturing the transformed mammalian cell in a protein production medium; and
collecting the produced fusion protein, wherein
the gene encoding the fusion protein contains the nucleotide sequence of a gene encoding the BDNF and the nucleotide sequence of a gene encoding the anti-transferrin receptor antibody or a fragment thereof, and
the chaperone protein includes one or more selected from the group consisting of HSP90α, HSP900, CDC37, HSP70, HSP40, HSP60, HSP10, HSP110, and HSP27.

### <Providing mammalian cell>

In this step, a mammalian cell is provided. For the mammalian cell, any of the mammalian cells shown as examples in "Method for producing fusion protein (1)" in the above can be used. That is, it is preferable that the mammalian cell include one or more selected from the group consisting of a CHO cell, a COS cell, a BHK cell, a HeLa cell, an HEK293 cell, an NS0 cell, and an Sp2/0 cell.

### <Transforming mammalian cell>

In this step, the mammalian cell is transformed with a gene encoding a fusion protein and a gene encoding a chaperone protein. The gene encoding the fusion protein contains the nucleotide sequence of a gene encoding the BDNF and the nucleotide sequence of a gene encoding the anti-transferrin receptor antibody or a fragment thereof.

For the fusion protein, any of the fusion proteins shown as examples in "Method for producing fusion protein (1)" in the above can be used.

That is, the fusion protein contains BDNF and an anti-transferrin receptor antibody or a fragment thereof. Examples of the fragment of the anti-transferrin receptor antibody include a Fab fragment, which consists of a heavy chain (H chain) fragment of an antibody and a light chain (L chain) fragment of an antibody, an Fc fragment in which the Fab fragment is added to an antibody constant region, a single-chain antibody (scFv), and a bispecific antibody (diabody).

For the chaperone protein, any of the chaperone proteins shown as examples in "Method for producing fusion protein (1)" in the above can be used. That is, the chaperone protein includes one or more selected from the group consisting of HSP90α, HSP90β, CDC37, HSP70, HSP40, HSP60, HSP10, HSP110, and HSP27. In an aspect of the present embodiment, it is preferable that the chaperone protein include any one of HSP90α, HSP90β, HSP40, and CDC37, or include both HSP90α, HSP90β, or HSP40 and CDC37. In another aspect of the present embodiment, it is preferable that the chaperone protein include either one or both of HSP90α and CDC37.

In the present embodiment, the order of introducing the gene encoding the fusion protein and the gene encoding the chaperone protein into the mammalian cell as a host cell is not limited. The gene encoding the fusion protein and then the gene encoding the chaperone protein may be introduced into the mammalian cell. The gene encoding the chaperone protein and then the gene encoding the fusion protein may be introduced into the mammalian cell. Alternatively, the gene encoding the fusion protein and the gene encoding the chaperone protein may be simultaneously introduced into the mammalian cell.

For example, the ratio of the gene encoding the fusion protein and the gene encoding the chaperone protein in introducing the two genes into the host cell may be 1:1 to 10:1, and more specifically 4:1.

In an aspect of the present embodiment, it is preferable that the transforming the mammalian cell is performed with one or more recombinant protein expression vectors each containing a gene encoding the fusion protein and a gene encoding the chaperone protein. Since the recombinant protein expression vectors each contain a gene encoding the fusion protein together with a gene encoding the chaperone protein, the recombinant protein expression vectors can be regarded as expression-enhancing vectors.

In this case, in each of the recombinant protein expression vectors, identical or different promoter sequences may be disposed in the upstream of the gene encoding the fusion protein and in the upstream of the gene encoding the chaperone protein. Construction of each recombinant protein expression vector in this manner allows expressions of the fusion protein and the chaperone protein to be individually controlled.

In another aspect of the present embodiment, in each of the recombinant protein expression vectors, a promoter sequence, the gene encoding the fusion protein, and the gene encoding the chaperone protein may be disposed in order from the 5'-end side, and a promoter sequence, the gene encoding the chaperone protein, and the gene encoding the fusion protein may be disposed in order from the 5'-end side. Construction of each recombinant protein expression vector in this manner allows expressions of the fusion protein and the chaperone protein to be simultaneously controlled with a single promoter sequence. The polypeptides expressed are inferred to be cleaved at proper sites to become the fusion protein and the chaperone protein.

In another aspect of the present embodiment, in the case that the additional protein is a dimer, the gene encoding the fusion protein may be composed of a first gene containing the nucleotide sequence of a gene encoding the BDNF and the nucleotide sequence of a gene encoding the first subunit constituting the additional protein, and a second gene containing the nucleotide sequence of a gene encoding the second subunit constituting the additional protein. In this case, it is preferable that the transforming the mammalian cell be performed with one or more recombinant protein expression vectors containing the first gene, the second gene, and a gene encoding the chaperone protein.

In another aspect of the present embodiment, it is preferable that the transforming the mammalian cell is performed by simultaneously or separately bringing one or more recombinant protein expression vectors each containing a gene encoding the fusion protein and one or more expression-enhancing vectors each containing a gene encoding the chaperone protein into contact with the mammalian cell.

In another aspect of the present embodiment, in the case that the additional protein is a dimer, it is preferable that the transforming the mammalian cell be performed by simultaneously or separately bringing a first recombinant protein expression vector containing the above first gene, a second recombinant protein expression vector containing the above second gene, and one or more expression-enhancing vectors each containing a gene encoding the chaperone protein into contact with the mammalian cell.

### <Producing fusion protein>

In this step, the fusion protein is produced by culturing the transformed mammalian cell in a protein production medium. For the specific method, the method described in "Method for producing fusion protein (1)" in the above can be used.

### <Collecting fusion protein>

In this step, the produced fusion protein is collected. For the specific method, the method described in "Method for producing fusion protein (1)" in the above can be used.

### «Mammalian cell for recombinant protein production»

A mammalian cell for recombinant protein production in the present embodiment is a mammalian cell for recombinant protein production containing one or more recombinant protein expression vectors each containing a gene encoding a fusion protein, wherein
the fusion protein contains BDNF and an anti-transferrin receptor antibody or a fragment thereof,
the gene encoding the fusion protein contains the nucleotide sequence of a gene encoding the BDNF and the nucleotide sequence of a gene encoding the anti-transferrin receptor antibody or a fragment thereof,
the mammalian cell for recombinant protein production further contains one or more expression-enhancing vectors each containing a gene encoding a chaperone protein, and
the chaperone protein includes one or more selected from the group consisting of HSP90α, HSP900, CDC37, HSP70, HSP40, HSP60, HSP10, HSP110, and HSP27.

### <<Kit for enhancing production of fusion protein>>

A kit in the present embodiment is a kit for enhancing the production of a fusion protein containing BDNF and an anti-transferrin receptor antibody or a fragment thereof in a mammalian cell, wherein
the kit includes one or more expression-enhancing vectors each containing a gene encoding a chaperone protein, and
the chaperone protein includes at least one selected from the group consisting of HSP90α, HSP900, CDC37, HSP70, HSP40, HSP60, HSP10, HSP110, and HSP27.

In the present embodiment, the kit may further include one or more selected from the group consisting of a buffer solution, a mammalian cell as a host cell, a recombinant protein expression vector, a protein production medium, a sample tube, a microplate, an instruction for users of the kit, and a transfection reagent.

### «Pharmaceutical composition»

A fusion protein produced by the production method of the present invention can be used as a raw material of a pharmaceutical composition containing the fusion protein as an active ingredient. The invention of the present application includes a method for producing the pharmaceutical composition, the method including bringing the fusion protein and an excipient into contact. For the excipient, any component commonly known as an excipient to be contained in pharmaceutical compositions can be appropriately selected without limitation.

### EXAMPLES

Hereinafter, examples according to the present invention will be described, but the present invention is not limited thereto.

### <<Preparation of mammalian expression plasmid (expression-enhancing vector) for expression-enhancing factors>>

The following nine genes were used as expression-enhancing factors (chaperone proteins) for examination.
(1) Human heat shock protein 90α (HSP90α) gene (HSP90AA1) (GenBank No. NP_001017963, amino acid sequence: SEQ ID NO: 2) (nucleotide sequence after codon optimization: SEQ ID NO: 1),
(2) Human Cell Division Cycle 37, HSP90 cochaperone (CDC37) gene (Genbank No. NP_008996, amino acid sequence: SEQ ID NO: 16) (nucleotide sequence after codon optimization: SEQ ID NO: 15),
(3) Human HSP60 gene (GenBank No. NP_955472, amino acid sequence: SEQ ID NO: 20) (nucleotide sequence after codon optimization: SEQ ID NO: 19),
(4) Human HSP10 gene (GenBank No. NP_002148, amino acid sequence: SEQ ID NO: 24) (nucleotide sequence after codon optimization: SEQ ID NO: 23),
(5) Human HSP110 gene (GenBank No. NP_006635, amino acid sequence: SEQ ID NO: 26) (nucleotide sequence after codon optimization: SEQ ID NO: 25),
(6) Human HSP40 gene (GenBank No. NP_001530, amino acid sequence: SEQ ID NO: 22) (nucleotide sequence after codon optimization: SEQ ID NO: 21),
(7) Human HSJ1 gene (GenBank No. AAA09034, amino acid sequence: SEQ ID NO: 34) (nucleotide sequence after codon optimization: SEQ ID NO: 33),
(8) HSP70 gene of Chinese hamster ovary-derived cell, CHO (J. Biotechnology 143 (2009) 34-43) (nucleotide sequence after codon optimization: SEQ ID NO: 27, amino acid sequence: SEQ ID NO: 28), and
(9) HSP27 gene of Chinese hamster ovary-derived cell, CHO (J. Biotechnology 143 (2009) 34-43) (nucleotide sequence after codon optimization: SEQ ID NO: 29, amino acid sequence: SEQ ID NO: 30).

For each of the nine genes, an optimum nucleotide sequence for an expression system with CHO cells was determined by using OptimumGene (codon optimization) from GenScript. For each of the nine genes, a Kozak sequence (ccacc) and a stop codon (TGA) were respectively added to the N terminus and C terminus of the determined optimum nucleotide sequence to produce a gene fragment through chemical synthesis. Each gene fragment was inserted into a HindIII-EcoRI site of a pcDNA3.1(+) vector (Cat. No. V79020, Invitrogen), which is an expression vector for mammals, to produce a plasmid vector for the expression-enhancing factor (1 mg/mL). Through the described steps, nine expression-enhancing vectors were obtained.

As a control to the expression-enhancing factors, an Enhanced Green Fluorescent Protein (EGFP) gene (GenBank No. AAF62891.1) was used. For the EGFP gene, an optimum nucleotide sequence for an expression system with CHO cells was determined by using OptimumGene (codon optimization) from GenScript. A Kozak sequence (ccacc) and a stop codon (TGA) were respectively added to the N terminus and C terminus of the determined optimum nucleotide sequence to produce a gene fragment through chemical synthesis. The gene fragment was inserted into a HindIII-EcoRI site of a pcDNA3.1(+) vector (Cat. No. V79020, Invitrogen), which is an expression vector for mammals, to produce a plasmid vector for the control (1 mg/mL).

### <<Preparation of mammalian expression plasmid (recombinant protein expression vector) for fusion protein consisting of BDNF and additional protein>>

The following genes were provided as genes encoding a fusion protein consisting of BDNF and an additional protein.
(1) First gene: hBDNF-hFab (H) gene (nucleotide sequence after codon optimization: SEQ ID NO: 39, amino acid sequence: SEQ ID NO: 40, here, hFab (H) is a Fab heavy chain of an anti-transferrin receptor antibody)
(2) Second gene: hFab (L) gene (nucleotide sequence after codon optimization: SEQ ID NO: 43, amino acid sequence: SEQ ID NO: 44, here, hFab (L) is a Fab light chain of an anti-transferrin receptor antibody)

The hBDNF-hFab (H) gene is a gene encoding a polypeptide consisting of, in order from the N-terminal side, an IgG signal sequence (GenBank No. 6SVL_B, nucleotide sequence after codon optimization: SEQ ID NO: 35, amino acid sequence: SEQ ID NO: 36), human BDNF (nucleotide sequence after codon optimization: SEQ ID NO: 31, amino acid sequence: SEQ ID NO: 32), a glycine linker (amino acid sequence: SEQ ID NO: 75), and a human anti-transferrin receptor antibody Fab H chain fragment (nucleotide sequence after codon optimization: SEQ ID NO: 37, amino acid sequence: SEQ ID NO: 38) as a first subunit.

The hFab (L) gene is a gene encoding a polypeptide consisting of, in order from the N-terminal side, an IgG signal sequence (GenBank No. 6SVL_B, nucleotide sequence after codon optimization: SEQ ID NO: 35, amino acid sequence: SEQ ID NO: 36) and a human anti-transferrin receptor antibody Fab L chain fragment (nucleotide sequence after codon optimization: SEQ ID NO: 41, amino acid sequence: SEQ ID NO: 42) as a second subunit.

For each of the two genes, an optimum nucleotide sequence for an expression system with CHO cells was determined by using OptimumGene (codon optimization) from GenScript. For each of the two genes, a Kozak sequence (ccacc) and a stop codon (TAA) were respectively added to the N terminus and C terminus of the determined optimum nucleotide sequence to produce a gene fragment through chemical synthesis. Each gene fragment was inserted into a HindIII-EcoRI site of a pcDNA3.1(+) vector (Cat. No. V79020, Invitrogen), which is an expression vector for mammals, to produce a plasmid vector for the recombinant protein (1 mg/mL). Through the described steps, two recombinant protein expression vectors were obtained.

### <<Examination of enhancing effects of expression-enhancing factors in production of a human BDNF-human anti-transferrin receptor antibody Fab fragment by using Expi-CHO expression system>>

The following operations were performed by using a Gibco (TM) Expi (TM) Expression System (Cat. No. A29133, ThermoFisher Scientific K.K.) in accordance with a Max Titer protocol. First, cultured Expi-CHO cells (6 × 10⁶ cells/mL) were added to a 125-mL Erlenmeyer flask (Corning Inc. Cat. No. 431143) containing ExpiCHO (TM) Expression Medium (Cat. No. A29100-01, ThermoFisher Scientific K.K.) (25 mL). Next, a reagent (1 ml) containing plasmid vectors shown in Table 1 below was prepared. Separately from the tube for the reagent containing plasmid vectors, Expifectamine (Cat. No. A12129) (80 µL) and OptiPRO (TM) SFM (Cat. No. 12309050) (920 µL) were added to another tube. The reagent containing plasmid vectors and the reagent containing Expifectamine were each stirred, and left to stand at room temperature for 5 minutes. Thereafter, the two reagents were slowly mixed together to form ExpiFectamine (TM) CHO/plasmid DNA complexes, which were left to stand at room temperature for 1 to 5 minutes. The complexes were added to the 125-mL Erlenmeyer flask containing Expi-CHO cells, and stirring culture was performed at 37°C, 8% CO₂, and 125 rpm overnight.

**[Table 1]**

| Sample No. | Fusion protein (plasmid name) | Chaperone protein or control (plasmid name) | SFM* (µL) | Total volume (µL) |
|---|---|---|---|---|
| 1-1 | pcDNA3.1 (+)hBDNF-hFab(H)(1 mg/mL) 8µL pcDNA3.1 (+)hFab(L)(1 mg/mL) 8 µL | pcDNA3.1(+)EGFP(1 mg/mL) 4µL | 980 | 1000 |
| 1-2 | same as above | pcDNA3.1(+)hHSP90AA1 _ NP _0 01017963 (1 mg/mL) 4 µL | 980 | 1000 |
| 1-3 | same as above | pcDNA3.1 (+)CH-HSP70 (1 mq/mL) 4 µL | 980 | 1000 |
| 1-4 | same as above | pcDNA3.1 (+)CH-HSP27 (1 mg/mL) 4 µL | 980 | 1000 |
| 1-5 | same as above | pcDNA3.1(+)hCDC37 (1 mg/mL) | 980 | 1000 |
| 1-6 | same as above | pcDNA3.1 (+)hHSP40 (1 mg/mL) 4 µL | 980 | 1000 |
| 1-7 | same as above | pcDNA3.1(+)hHSP10 (1 mg/mL) 4 µL | 980 | 1000 |
| 1-8 | same as above | pcDNA3.1(+)HSP110 (1 mg/mL) 4 µL | 980 | 1000 |
| 1-9 | same as above | pcDNA3.1(+)EGFP(1 mg/mL) 2 µL | 980 | 1000 |
| | | pcDNA3.1(+)hHSP90AA1 _ NP _0 01017963 (1 mg/mL) 2 µL | | |
| 1-10 | same as above | pcDNA3.1 (+)hHSP90AA1 _ NP _0 01017963 (1 mg/mL) 2 µL | 980 | 1000 |
| | | pcDNA3.1(+)hCDC37 (1 mg/mL) 2 µL | | |
| 1-11 | same as above | pcDNA3.1 (+)hHSP90AA1 _ NP _0 01017963 (1 mg/mL) 2 µL | 980 | 1000 |
| | | pcDNA3.1 (+)hHSP40 (1 mg/mL) 2 µL | | |
| 1-12 | same as above | pcDNA3.1 (+)hHSP90AA1_NP _0 01017963 (1 mg/mL) 2 µL | 980 | 1000 |
| | | pcDNA3.1 (+)CH-HSP70 (1 mg/mL) 2 µL | | |
| 1-13 | same as above | pcDNA3.1(+)HSP60 (1 mg/mL) 4 µL | 980 | 1000 |
| 1-14 | same as above | pcDNA3.1(+)HSJ1 (1 mg/mL) 4 µL | 980 | 1000 |

| | | | | |
|---|---|---|---|---|
| * OptiPRO (TM) SFM (Cat. No. 12309050) | | | | |

Within 18 to 22 hours after transfecting with the plasmid vectors, ExpiFectamine (TM) CHO Enhancer (150 µL) and ExpiCHO (TM) Feed (4 mL) were added to each of the culture solutions, and culture was performed at 32°C, 5% CO₂, and 125 rpm. On day 5 of culture, ExpiCHO (TM) Feed (4 mL) was further added to each of the culture solutions, and culture was performed at 32°C, 5% CO₂, and 125 rpm. Between day 7 and day 13 of culture, the culture supernatants were collected, and the productivities for the human BDNF-human anti-transferrin receptor antibody Fab fragment (hereinafter, occasionally referred to as "fusion protein" or "BDNF-hFab") were calculated through ELISA. The cell survival rates were calculated through measurement of total cell counts and viable cell counts by using a Countess II FL automatic cell counter (Cat. No. AMQAF1000, ThermoFisher Scientific K.K.).

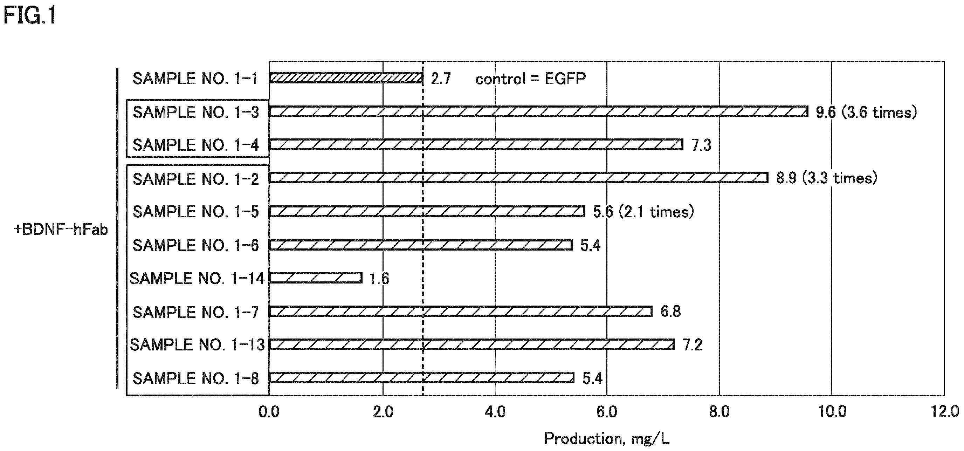

The fusion protein concentrations of the culture supernatants were calculated by using ELISA (Biosensis Pty Ltd., Cat. No. BEK-2211-1P/2P). On day 13 of culture, cells were collected, and the viable cell counts were determined by using a Countess II FL automatic cell counter, centrifugation was performed at 10,000 ×g for 5 minutes, and the culture supernatants were then collected. The culture supernatants collected were diluted to a degree that allowed quantification with standards (7.8 to 500 pg/mL) (dilution rate: 30,000-fold to 1,000,000-fold). To wells of a microplate, standards, a QC sample included in the kit, and the diluted culture supernatants each in 100 µL were added, and stirring was performed at room temperature for 45 minutes. Thereafter, the samples were removed from the wells, and the wells were washed five times with a washing solution (200 µL/well × 5 times). A detection antibody was added at 100 µL/well, and stirring was performed at room temperature for 30 minutes. The antibody was removed from the wells, and washing was performed in the same manner. Tetramethylbenzidine reagent (TMB reagent) was added to the wells for reaction, and, after finding the occurrence of reaction with moderate blue coloring, a stop solution was added to the wells. The absorbances at a wavelength of 450 nm were measured by using a microplate reader (SpectraMax M5e, Molecular Devices LLC.), and the production levels (concentrations) of fusion protein in the culture supernatants were calculated from the standards to determine production-enhancing effects depending on the presence or absence of expression-enhancing factors (Fig. 1). The results found that HSP90α (sample 1-2) and CHO-derived HSP70 (sample No. 1-3) enhanced the production of the fusion protein the most. Furthermore, CHO-derived HSP27 (sample No. 1-4), human CDC37 (sample No. 1-5), HSP40 (sample No. 1-6), HSP10 (sample No. 1-7), and HSP110 (sample No. 1-8) were also found to have production-enhancing effect for the fusion protein. By contrast, HSJ1 (sample No. 1-14) did not exhibit such effect at all.

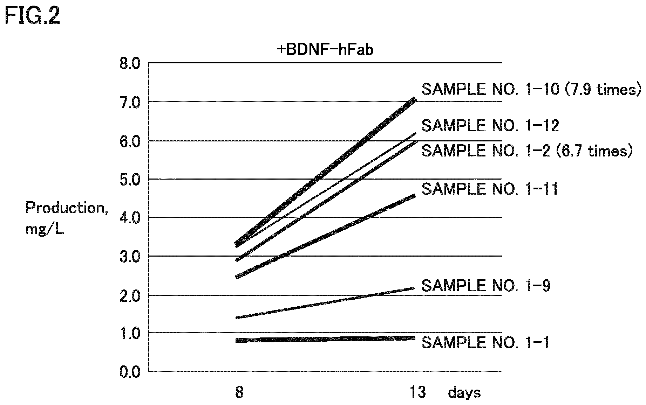

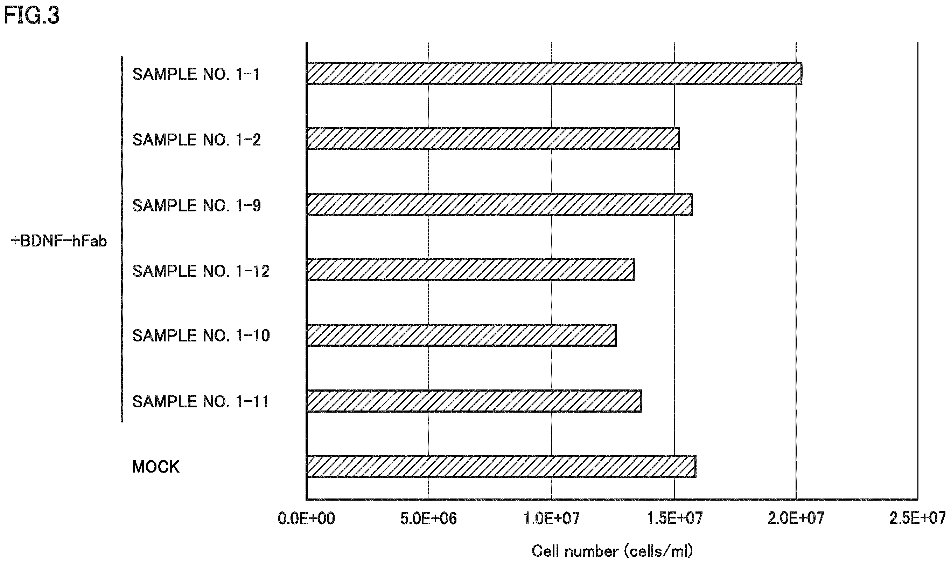

Next, for CDC37, which has been known to form a chaperone with HSP90α in cells, the effect was checked (Fig. 2). It was found that co-expression with CDC37 resulted in higher production-enhancing effect for the fusion protein than in the case of single expression of HSP90α (sample No. 1-2 and sample No. 1-10 in Fig. 2). It was revealed that allowing the fusion protein to be expressed in Expi-CHO causes no change in cell growth, and co-expression with a chaperone protein such as HSP90α causes no change in cell growth (Fig. 3).

Although embodiments and examples of the present invention have been described as above, appropriate combinations of the configurations of the embodiments and examples described above have been originally contemplated.

The embodiments and examples disclosed herein are exemplary in all respects, and should not be construed to be restrictive. The scope of the present invention is specified not by the above embodiments and examples but by claims, and modifications equivalent in meaning to claims and those within the scope of claims are all intended to be included.

## Claims

1. A method for producing a fusion protein containing BDNF and an anti-transferrin receptor antibody or a fragment thereof, the method comprising:
producing the fusion protein by culturing a transformed mammalian cell containing a gene encoding the fusion protein and a gene encoding an exogeneous chaperone protein in a protein production medium; and
collecting the produced fusion protein, wherein
the gene encoding the fusion protein contains a nucleotide sequence of a gene encoding the BDNF and a nucleotide sequence of a gene encoding the anti-transferrin receptor antibody or a fragment thereof, and
the chaperone protein includes one or more selected from the group consisting of HSP90α, HSP90β, CDC37, HSP70, HSP40, HSP60, HSP10, HSP110, and HSP27.

2. The method for producing a fusion protein containing the BDNF and the anti-transferrin receptor antibody or a fragment thereof according to claim 1, the method comprising:
providing a mammalian cell;
transforming the mammalian cell with a gene encoding the fusion protein and a gene encoding the chaperone protein;
producing the fusion protein by culturing the transformed mammalian cell in a protein production medium; and
collecting the produced fusion protein, wherein
the gene encoding the fusion protein contains a nucleotide sequence of a gene encoding the BDNF and a nucleotide sequence of a gene encoding the anti-transferrin receptor antibody or a fragment thereof, and
the chaperone protein includes one or more selected from the group consisting of HSP90α, HSP90β, CDC37, HSP70, HSP40, HSP60, HSP10, HSP110, and HSP27.

3. The method for producing a fusion protein according to claim 2, wherein the transforming the mammalian cell is performed with one or more recombinant protein expression vectors each containing a gene encoding the fusion protein and a gene encoding the chaperone protein.

4. The method for producing a fusion protein according to claim 2, wherein the transforming the mammalian cell is performed by simultaneously or separately bringing one or more recombinant protein expression vectors each containing a gene encoding the fusion protein and one or more expression-enhancing vectors each containing a gene encoding the chaperone protein into contact with the mammalian cell.

5. The method for producing a fusion protein containing the BDNF and the anti-transferrin receptor antibody or a fragment thereof according to claim 1, the method comprising:
providing a mammalian cell containing one or more recombinant protein expression vectors each containing a gene encoding the fusion protein;
transforming the mammalian cell with at least one expression-enhancing vector containing a gene encoding the chaperone protein;
producing the fusion protein by culturing the transformed mammalian cell in a protein production medium; and
collecting the produced fusion protein, wherein
the gene encoding the fusion protein contains a nucleotide sequence of a gene encoding the BDNF and a nucleotide sequence of a gene encoding the anti-transferrin receptor antibody or a fragment thereof, and
the chaperone protein includes one or more selected from the group consisting of HSP90α, HSP90β, CDC37, HSP70, HSP40, HSP60, HSP10, HSP110, and HSP27.

6. The method for producing a fusion protein according to claim 5, wherein
the expression-enhancing vector includes a first expression-enhancing vector containing a gene encoding a first chaperone protein and a second expression-enhancing vector containing a gene encoding a second chaperone protein, and
the first chaperone protein is different from the second chaperone protein.

7. The method for producing a fusion protein according to any one of claims 1 to 6, wherein the chaperone protein includes either one or both of HSP90α and CDC37.

8. The method for producing a fusion protein according to any one of claims 1 to 7, wherein the BDNF is bound, directly or via a linker peptide, to the anti-transferrin receptor antibody or a fragment thereof.

9. The method for producing a fusion protein according to claim 8, wherein the linker peptide contains an amino acid sequence selected from the group consisting of Gly, Ser, Gly-Ser, Gly-Gly-Ser, Gly-Gly-Gly-Gly-Ser, Gly-Gly-Gly-Gly-Gly-Ser, Ser-Gly-Gly-Gly-Gly, and any 1 to 10 consecutive amino acid sequences selected from those amino acid sequences.

10. The method for producing a fusion protein according to any one of claims 1 to 9, wherein the BDNF contains an amino acid sequence having a sequence identity of at least 90% with an amino acid sequence set forth in SEQ ID NO: 32.

11. The method for producing a fusion protein according to any one of claims 1 to 10, wherein the fragment of the anti-transferrin receptor antibody is a Fab fragment, a F(ab')₂ fragment, or a F(ab') fragment.

12. The method for producing a fusion protein according to any one of claims 1 to 11, wherein the mammalian cell includes one or more selected from the group consisting of a CHO cell, a COS cell, a BHK cell, a HeLa cell, an HEK293 cell, an NS0 cell, and an Sp2/0 cell.

13. A mammalian cell for recombinant protein production, comprising one or more recombinant protein expression vectors each containing a gene encoding a fusion protein, wherein
the fusion protein contains BDNF and an anti-transferrin receptor antibody or a fragment thereof,
the gene encoding the fusion protein contains a nucleotide sequence of a gene encoding the BDNF and a nucleotide sequence of a gene encoding the anti-transferrin receptor antibody or a fragment thereof,
the mammalian cell for recombinant protein production further comprises one or more expression-enhancing vectors each containing a gene encoding a chaperone protein, and
the chaperone protein includes one or more selected from the group consisting of HSP90α, HSP90β, CDC37, HSP70, HSP40, HSP60, HSP10, HSP110, and HSP27.

14. A kit for enhancing a production of a fusion protein containing BDNF and an anti-transferrin receptor antibody or a fragment thereof in a mammalian cell, wherein
the kit comprises one or more expression-enhancing vectors each containing a gene encoding a chaperone protein, and
the chaperone protein includes at least one selected from the group consisting of HSP90α, HSP90β, CDC37, HSP70, HSP40, HSP60, HSP10, HSP110, and HSP27.
